# FASCICULE DE BREVET EUROPEEN

(11) **EP 2 882 413 B1**
(45) Date de publication et mention de la délivrance du brevet: **11.04.2018**
(21) Numéro de dépôt: 13756640.2
(22) Date de dépôt: 31.07.2013
(51) Int. Cl.: A61K 8/49, A61K 8/60, A61Q 17/00, A61Q 19/00

(54) **INHIBITION DE L'ADHESION DE MICRO-ORGANISMES PATHOGENES PAR UN SUCROSE STEARATE DANS LE TRAITEMENT COSMETIQUE DE L'ATOPIE CUTANEE**
HEMMUNG DER ADHÄSION VON PATHOGENEN MIKROORGANISMEN DURCH EIN SACCHAROSESTEARAT IN DER KOSMETISCHEN BEHANDLUNG VON KUTANER ATOPIE
INHIBITION OF THE ADHESION OF PATHOGENIC MICROORGANISMS BY A SUCROSE STEARATE IN THE COSMETIC TREATMENT OF CUTANEOUS ATOPY

(30) Priorité: 07.08.2012 FR 1257681
(43) Date de publication de la demande: 17.06.2015
(60) Demande divisionnaire: 17180846.2
(73) Titulaire: Thorel, Jean-Noël, 75014 Paris (FR)
(72) Inventeur: THOREL, Jean-Noël, F-75014 Paris (FR); GATTO, Hugues, F-06570 Saint Paul de Vence (FR)
(74) Mandataire: Cabinet Laurent & Charras
(86) Numéro de dépôt international: PCT/FR2013/051856
(87) Numéro de publication internationale: WO 2014/023895

(56) Documents cités:
- EP-A1- 0 815 841
- EP-A1- 1 639 989
- EP-A1- 2 210 588
- EP-A2- 0 875 239
- WO-A1-02/098412
- WO-A2-2004/037225
- FR-A1- 2 798 591
- FR-A1- 2 970 177
- US-A1- 2005 158 348
- US-A1- 2010 080 768
- DATABASE GNPD [Online] MINTEL; mars 2010 (2010-03), "Exomega DM Balm", XP002721070, Database accession no. 1291195
- DATABASE GNPD [Online] MINTEL; July 2011 (2011-07), Database accession no. 1593107

## Description

### DOMAINE DE L'INVENTION

L'invention a pour objet un procédé de traitement cosmétique de l'atopie cutanée humaine par le biais d'un revêtement de la surface cutanée au moyen d'une composition cosmétique formant un film sur la peau. Selon l'invention, ce film, de par les constituants qu'il contient, permet à la fois d'inhiber l'adhésion de microorganismes pathogènes sur la peau, et de renforcer l'intégrité de la barrière cutanée.

Plus précisément, la composition cosmétique comprend un ester de saccharose et/ou un ester de sorbitan apte à empêcher l'adhésion sur la peau et la muqueuse nasale de *Staphylococcus aureus* et donc la prolifération de la flore pathogène. Cette inhibition a en outre pour effet de protéger la barrière cutanée en limitant la dégradation des lipides, en particulier des céramides, dont *Staphylococcus aureus* est indirectement responsable.

### ETAT DE LA TECHNIQUE

L'atopie cutanée, ou dermatite atopique, est une dermatose inflammatoire chronique prurigineuse liée à une prédisposition héréditaire du système immunitaire qui s'accompagne d'anomalies de la barrière cutanée. Cliniquement, cette prédisposition héréditaire se manifeste aussi par, notamment, une rhinite allergique et de l'asthme.

L'atopie cutanée se traduit par une hypersensibilité à des allergènes de l'environnement qui sont normalement tolérés chez des sujets sains.

L'eczéma au cours de la dermatite atopique représente une forme de réaction d'hypersensibilité retardée qui met en jeu des lymphocytes de type Th2 producteurs notamment d'IL-4 et d'IL-5 et des cellules présentatrices d'antigènes.

Comme toute réaction immunitaire liée à des lymphocytes T spécifiques d'antigènes, la réaction inflammatoire de l'eczéma de la dermatite atopique comporte 3 phases.

La première phase est une sensibilisation asymptomatique. Cette phase de sensibilisation est cliniquement muette et aboutit à la génération de lymphocytes T spécifiques. La sensibilisation se fait classiquement pendant la petite enfance par pénétration des allergènes de l'environnement, captés par les cellules dendritiques.

Dans une deuxième phase, le déclenchement des lésions d'eczéma se produit. Après un nouveau contact avec les allergènes, les cellules de Langerhans vont, après migration, activer des lymphocytes Th2 spécifiques producteurs de cytokines, qui vont à leur tour activer différents types cellulaires au niveau cutané. Ce mécanisme, contribue au recrutement des leucocytes au niveau du derme et de l'épiderme, ces derniers étant responsables de la production de médiateurs inflammatoires. Cette phase de durée plus ou moins longue se caractérise par les lésions d'eczéma. Elle s'accompagne chez la majorité des patients de taux élevés d'IgE spécifiques d'allergènes et de leur expression à la surface des cellules de Langerhans.

Enfin, dans une troisième phase on observe la résolution des lésions. L'eczéma de la dermatite atopique évolue par poussées entrecoupées de rémissions spontanées dont les mécanismes mis enjeu dans la régulation de l'inflammation restent très mal connus.

L'atopie cutanée toucherait entre 10 et 30% de la population, serait en constante augmentation dans les pays industrialisés, et aurait même été multipliée par 2 ou 3 durant les 20 dernières années.

L'atopie cutanée est caractérisée par des poussées prurigineuses d'eczéma aïgue, entrecoupées de périodes de rémission et concerne surtout les enfants. Elle se manifeste quelques mois après la naissance par des lésions sur les joues et les zones de frottements. Elle évolue ensuite par crises, entre 1 et 2 ans, période durant laquelle la peau est sèche, faisant apparaitre des plaques rouges suintantes, surtout dans les plis de flexion. A partir de l'âge de 5 ans, les crises ont tendance à disparaître, mais la peau demeure sèche et sensible. Dans certains cas, l'atopie cutanée peut persister à l'âge adulte.

Deux phénomènes sont entretenus par l'atopie cutanée. Il s'agit d'une part de l'altération de la barrière cutanée, et d'autre part de la colonisation de la peau par une flore de microorganismes pathogènes.

En effet, la fonction de barrière cutanée est tout d'abord assurée par le *stratum corneum,* composé de cellules appelées cornéocytes, et de lipides spécifiques comme le cholestérol, des acides gras libres, des cérébrosides et des céramides. Ces lipides jouent le rôle de ciment intercellulaire, assurant ainsi l'étanchéité du *stratum corneum.*

Une analyse du *stratum corneum* de patients atteints d'atopie cutanée montre une déficience marquée en certaines protéines et certains lipides cutanés. On observe une déficience en filaggrine, protéine impliquée dans l'agrégation des filaments de kératine dans les cornéocytes et en involucrine, protéine essentielle à la constitution du squelette protéique de l'enveloppe cornée. En ce qui concerne les lipides, ce sont notamment un déficit en céramides 1 et 3 qui est observé.

Les céramides jouent un rôle particulièrement important dans la régulation de la fonction de barrière de la peau, en permettant de limiter l'évaporation de l'eau.

Ces déficits en protéines et lipides font que la cohésion des cornéocytes est diminuée, ce qui entraîne une perte de la cohésion et de l'étanchéité du *stratum corneum.* Ceci se traduit par une sensible réduction de l'épaisseur du *stratum corneum* chez ces patients.

L'atopie cutanée se manifestant par une perte significative en protéines et lipides cutanées, comme vu plus haut, il s'ensuit une évaporation de l'eau contenue dans la peau, et donc une sécheresse cutanée. Celle-ci est par ailleurs connue pour favoriser la pénétration d'allergènes à travers la peau.

L'atopie cutanée engendre donc un cercle vicieux : l'évaporation de l'eau intracellulaire aboutit à une déshydratation cutanée et à une sécheresse de la peau, et donc à une augmentation de la perméabilité cutanée. L'augmentation de la perméabilité cutanée favorise la pénétration d'allergènes à travers la peau, qui à leur tour entretiennent la réactivité cutanée.

La fonction de barrière de la peau est aussi assurée par un écosystème constitué par une flore bactérienne saprophyte.

Ainsi, la flore bactérienne saprophyte est naturelle et permanente à la surface de la peau. Le nombre de bactéries à la surface de la peau est estimé entre 10² et 10⁵/cm². Les espèces les plus représentées sont des staphylocoques, notamment *Staphyloccocus epidermidis,* à coagulase négative et apparenté (*Micrococcus*) et les corynéformes aérobies (*Corynebacterium, Brevibacterium*) ou anaérobies (*Propionibacterium acnes*). Ces bactéries sont établies sur le *stratum corneum,* où elles adhèrent aux cornéocytes, formant un biofilm protecteur.

Cette flore bactérienne saprophyte joue donc un rôle de barrière puisqu'elle occupe les sites d'adhésion d'autres microorganismes, éventuellement pathogènes, limitant ainsi leur prolifération.

Cependant, en cas d'altération de la peau, comme dans le cas de l'atopie cutanée, l'altération de la barrière cutanée favorise l'évaporation de l'eau créant un environnement de surface propice à des infections bactériennes, virales et/ou fongiques de la peau. Cet environnement propice est renforcé par l'augmentation de la température cutanée, survenant lors de la crise.

C'est ainsi que peut se développer, de façon transitoire, à la surface de la peau, une flore bactérienne pathogène. La plupart de ces bactéries niche dans l'environnement, comme certaines espèces des genres *Pseudomonas* et *Acinetobacter,* ou alors appartient à la flore digestive ou buccale, comme les entérobactéries, les streptocoques, ou les bactéries du genre *Clostridium.*

Ces bactéries sont normalement incapables de proliférer à la surface d'une peau saine, mais peuvent être toutefois la source d'une infection en cas d'une altération de la barrière cutanée, comme c'est le cas pour l'atopie cutanée, et former notamment un biofilm bactérien pathogène.

Parmi les bactéries de la flore pathogène transitoire, le staphylocoque doré, ou *Staphylococcus aureus* (*S. aureus*) est l'espèce potentiellement la plus pathogène et la plus répandue du genre *Staphylococcus* et on la retrouve chez 15 à 30% des individus sains au niveau des fosses nasales et de la gorge. Cette bactérie à Gram positif colonise la peau en adhérant aux cellules cutanées.

Des études récentes ont montré que 90% des patients atteints d'atopie cutanée font l'objet d'une colonisation de la peau par le pathogène *S. aureus*, alors qu'une telle colonisation n'est observée que chez 5% des sujets sains

De plus, et de manière remarquable, l'atopie cutanée est aussi un facteur de risque avéré de la colonisation de *S. aureus* au niveau de la muqueuse nasale, qui constitue un réservoir microbien dans l'environnement des patients atopiques (Pascolini *et al.,* 2011).

Certaines études montrent que des céramidases produites par la flore bactérienne cutanée seraient activées par la présence de *S. aureus.* Les céramidases hydrolysent les céramides présents dans *stratum corneum,* ce qui suggère que la colonisation de la peau par *S. aureus* est en partie responsable du déficit en céramides observé dans l'épiderme des patients atopiques (Kita *et al.,* 2002).

*S. aureus* est par ailleurs à l'origine de superantigènes (toxines protéiques) qui, chez des sujets atteints d'atopie cutanée, entrent en interaction avec les cellules du système immunitaire, amplifient la réponse inflammatoire et favorisent ainsi le déclenchement des crises.

Il existe donc un lien tangible entre, d'une part, la prolifération de *S. aureus* à la surface de l'épiderme et d'autre part, la dégradation de la fonction barrière de l'épiderme par un déficit en céramides notamment chez les individus atopiques, étant rappelé que ladite fonction barrière participe aux phénomènes de sensibilisation aux allergènes de l'environnement et à l'entretien de l'inflammation cutanée.

En ce qui concerne les infections virales, le HSV (Herpes Simplex Virus) et le vaccinia virus sont les deux virus majeurs. De plus, il a aussi été observé une colonisation par la levure *Malassezia* chez des patients atteints d'atopie cutanée.

Actuellement, les traitements de l'atopie cutanée sont basés soit sur la lutte contre l'inflammation, soit sur la lutte contre la colonisation bactérienne.

L'administration de glucocorticostéroïdes et d'inhibiteurs de la calcineurine, comme le pimecrolimus et le tacrolimus, pour traiter l'inflammation a déjà été décrite. Il est préconisé d'associer des émollients à ces traitements.

La lutte contre l'infection bactérienne a été abondamment documentée, et a été notamment réalisée par le biais de l'administration d'antibiotiques à large spectre ou l'administration de solutions antiseptiques. Le premier traitement devient problématique à cause des phénomènes de résistances des souches bactériennes aux antibiotiques. De plus, les deux types de traitement visent à tuer les bactéries pathogènes présentes sur la peau, mais éliminent en même temps les bactéries saprophytes.

Ces traitements ont donc pour principal inconvénient d'éliminer un composant nécessaire à la fonction de barrière de la peau, à savoir la flore bactérienne saprophyte.

Des études récentes ont montré que l'application topique de compositions contenant certains sucres contribue à diminuer l'adhésion des bactéries pathogènes sur la peau.

Le document WO 2006/106220 décrit que certains sucres comme des mono- et oligosaccharides, tels que le rhamnose, le galactose, le mannose et le lauryl glucoside, ont un rôle négatif sur l'adhésion bactérienne, notamment du genre *Staphylococcus* (*S. intermedius*), sur des cornéocytes de chien atopique.

Le document WO 96/23479 suggère, quant à lui, l'utilisation de certains sucres comme agents inhibant l'adhésion de certains microorganismes. Sont notamment cités des monosaccharides, comme par exemple le raffinose, le mannose, le rhamnose, des disaccharides, des oligosaccharides, des sucres aminés et des esters de sucre, notamment des esters de glucose, comme par exemple le cétéarylglucoside, le caprylglucoside, le décylglucoside. Ces différents sucres peuvent rentrer, sous la forme d'un mélange, dans la composition de préparations cosmétiques ou dermatologiques, comme agents d'inhibition de l'adhésion bactérienne, comme par exemple *S. aureus*, et/ou pour le traitement, notamment de l'atopie cutanée.
Le document EP0875239A2 liste différents esters de sucre parmi lesquels les esters de saccharose pour leur activité vis-à-vis d'un certain nombre de bactéries Gram⁺ telles que *staphylococcus epidermidis, staphylococcus aureus, corynebacterium* ou encore *propionibacterium,* impliquées dans une liste de pathologies parmi lesquelles figurent la dermatite atopique. Les esters listés sont les diesters palmitate/stearate, les diesters stéarate, monoester laurate, les monoester myristate, les triesters stéarates et les tétraesters Cependant, dans les exemples de D1 on utilise un sucrose stéarate contenant seulement 70% en poids de monoesters. Le document EP1340486A1 liste différents esters de sucre parmi lesquels les esters de saccharose, de fructose, de glucose, de trehalose. Seuls les esters de fructose, de glucose et de trehalose sont testés pour leur capacité à inhiber la croissance de *staphylococcus aureus.* Les esters de sucrose ne sont pas testés pour cet effet. Le seul représentant de cette famille, en l'espèce le sucrose stearate est testé uniquement pour des propriétés blanchissantes (voir tableau 7). Enfin, ce document ne concerne pas la dermatite atopique.
Le document EP0815841A1 décrit une composition pour lutter contre les rougeurs de la peau, dues aux frottements des couches chez le nourrisson. Il ne s'agit pas du traitement de la dermatite atopique telle que décrite ci-avant. Ce document décrit l'effet inhibiteur de la croissance de *Staphylococcus aureus* et *de Staphylococcus epidermidis* d'un mélange de monoesters de sucrose avec de l'acide palmitique et de l'acide stéarique ou d'un mélange de monoesters avec de l'acide palmitique et de l'acide laurique.
Le document EP2210588A1 décrit des compositions moussantes contenant du polysorbate 80 c'est-à-dire, un ester de sorbitan. Le polysorbate 80 est utilisé classiquement en tant qu'agent émulsifiant.

Le document mintel avec numéro d'identification 1766174 décrit une composition hydradante contenant du sucrose stearate.
Le document WO4/037225A2 décrit des compositions sans alcool et sans propylène glycol servant de véhicule pour des aérosols moussants. La dermatite atopique est décrite parmi plusieurs pathologies susceptibles d'être traitées par l'aérosol après y avoir rajouté un actif spécifique. Dès lors, le sucrose stearate et le polysorbate 80 figurant dans les exemples sont utilisés classiquement comme agent tensio-actif.
Le document FR2798591A1 décrit l'utilisation d'huile végétale spécifique pour augmenter la synthèse des lipides cutanés, en particulier pour le traitement de la dermatite atopique. Sucrose distearate et sorbitan tristearate sont classiquement utilisés en tant qu'agents tensio-actifs.

Le document MERCK Chimie SAS 548.01.02.10 avec pour titre "Un coup de frais contre les rides" décrit une composition anti-rides comprenant du sucrose stearate.
Le document EP1639989A1 décrit la fabrication de micro émulsions comprenant des esters de sucre ou de sorbitan utilisés comme agents tensio-actifs.

Le document US2005/158348A1 décrit dans ses exemples 5 et 8, des compositions comprenant respectivement du polysorbate 80 ou encore un sucrose ester. Les compositions sont destinées au traitement de la douleur et de l'inflammation. Ester de sorbitan et ester de sucrose sont utilisés comme excipient.

Le document US2010/080768A1 décrit la présence de différents céramides et sphingolipides dans des compositions destinées au traitement des différentes pathologies dont la dermatite atopique.
Le document US2011/101135 décrit la mise en oeuvre de monocaprylate de sorbitan comme agent de conservation dans une composition cosmétique. Différentes souches bactériennes sont testées dont *staphylococcus aureus.*

Il existe néanmoins un besoin de trouver des alternatives plus efficaces aux différents traitements proposés jusqu'à présent pour lutter contre l'atopie cutanée.

C'est dans ce cadre que le Demandeur a découvert, de manière surprenante et inattendue, que certains esters de sucre, comme les esters de saccharose et les esters de sorbitan, présentent une forte inhibition de l'adhésion de microorganismes pathogènes sur la peau et la muqueuse nasale.

La diminution de l'adhésion de *S. aureus* à la surface de la peau prévient ainsi la prolifération des microorganismes pathogènes, et ce même en l'absence d'antibiotique, ou d'antiseptique topique.

Un des avantages de la diminution de l'adhésion de microorganismes pathogènes à la surface de la peau, en particulier *S. aureus*, est de maintenir une flore bactérienne saprophyte, et de renforcer la barrière cutanée en limitant la dégradation des lipides qu'elle contient.

Les esters de sucre peuvent être avantageusement combinés à des lipides naturellement présents dans l'épiderme pour former ainsi un film permettant de lutter davantage encore contre la dégradation de la barrière épidermique et la colonisation de la peau par des pathogènes.

D'autres buts et aspects avantageux de l'invention ressortiront mieux à la lecture de la description détaillée qui suit, donnée à titre indicatif et nullement limitatif.

### DESCRIPTION DETAILLEE DE L'INVENTION

Un premier aspect de l'invention a pour objet une composition cosmétique pour application topique comprenant au moins un ester de saccharose comme agent inhibant l'adhésion de *Staphylococcus aureus* sur la peau et la muqueuse nasale humaine.

Plus précisément, l'invention concerne une composition pour application topique comprenant au moins un ester de saccharose pour utilisation dans le traitement thérapeutique de l'atopie cutanée, comme agent inhibant l'adhésion de *Staphylococcus aureus* sur la peau et la muqueuse nasale humaine.

Les esters de sucre sont constitués par un sucre, dont au moins une fonction alcool libre est estérifiée par une chaîne d'acide gras.

Parmi les esters de sucre, plusieurs familles ont été décrites. Il s'agit notamment des esters de glucose, des esters de saccharose et des esters de sorbitan (Piccicuto *et al.,* 2001). Ils ont l'avantage d'être non toxiques et non irritants, et sont donc largement utilisés dans l'industrie alimentaire, pharmaceutique et cosmétique, en large partie pour leurs propriétés tensioactives.

Parmi ces 3 familles distinctes, les esters de saccharose font l'objet de la présente demande.

Ce sont donc des tensioactifs non ioniques, possédant un groupement hydrophile, constitué par le saccharose, relié par au moins une liaison ester à un groupement hydrophobe, constitué par un acide gras. Les propriétés tensioactives de ces esters permettent en outre de les combiner facilement avec des lipides dans les formulations cosmétiques de type notamment émulsion.

L'α-D-glucopyranosyl-β-D-fructofuranoside, ou saccharose, ou encore sucrose, possède 8 fonctions alcool libres et peut donc être estérifié par au maximum huit molécules d'acide gras.

Les chaînes carbonées des acides gras peuvent être plus ou moins longues. Par exemple, on pourra citer l'acide laurique, l'acide myristique, l'acide palmitique et l'acide stéarique, de chaîne carbonée C₁₂, C₁₄, C₁₆ et C₁₈ respectivement. L'acide gras est aussi choisi parmi les acides gras saturés et les acides gras insaturés, comme par exemple l'acide oléique (chaîne carbonée en C₁₈).

Ainsi, selon une autre caractéristique de l'invention, la composition cosmétique comprend du saccharose dont au moins une fonction alcool est estérifiée par un acide gras choisi dans le groupe des acides gras ayant une chaîne carbonée comprise entre C₁₂ et C₂₂, avantageusement entre C₁₂ et C₁₈.

En fonction de la quantité d'acides gras par rapport au saccharose, l'estérification peut aboutir à la formation de monoester, diester, triester ou polyester (au maximum un octaester), ainsi qu'à un mélange de toutes ces formes.

Selon la présente demande, la composition cosmétique comprend du saccharose dont toutes ou partie des fonctions alcool sont estérifiées par un acide gras identique.

Dans ce cas, et de manière avantageuse, l'ester de saccharose sera choisi dans le groupe comprenant le sucrolaurate, le sucromyristate, le sucropalmitate, le sucrostéarate.

Dans un mode de réalisation avantageux, l'ester de saccharose est un sucrose stéarate comprenant au moins 70%, avantageusement au moins 75% en poids de monoesters de sucrose et d'acide stéarique, le complément étant composé de di, tri, polyesters de sucrose et d'acide stéarique.

Selon la présente demande, la composition cosmétique peut être mise en oeuvre par du saccharose dont toutes ou partie des fonctions alcool sont estérifiées par au moins deux acides gras différents.

Dans ce cas, et de manière avantageuse, l'ester de saccharose sera choisi dans le groupe comprenant le sucropalmitate stéarate, le sucrotétrastéarate triacétate

Selon un mode de réalisation préféré, l'ester de saccharose dans la composition cosmétique selon l'invention est le sucrostéarate, c'est-à-dire une molécule de saccharose estérifiée par au moins une molécule d'acide stéarique (acide gras en C₁₈).

Il est connu de l'homme du métier que pour une longueur de chaîne carbonée de l'acide gras, la proportion en monoesters dans le mélange influence les propriétés hydrophile/lipophile de l'ester de sucre.

Ces propriétés sont caractérisées au moyen de l'échelle dite « HLB », pour « Hydrophily/Lipophily Balance », ou balance hydrophile/lipophile. Une valeur comprise entre 1 et 10 indique un ester de sucre à tendance lipophile, ce qui correspond à une proportion relative de monoesters faible et donc une proportion de polyesters élevée. En revanche, une valeur comprise entre 11 et 20 indique un ester de sucre à tendance hydrophile, correspondant à une proportion en monoesters élevée, par conséquent à une proportion en polyesters faible.

Dans un mode de réalisation particulier de l'invention, le sucrostéarate possède une valeur HLB de 16, ce qui correspond à une proportion relative en monoesters de sucrose et d'acide stéarique comprise entre 75 et 80%. Ce produit est désigné par la suite sucrose monostéarate.

De préférence, l'ester de saccharose représente 0,1% à 5% en poids de la composition, avantageusement entre 1 et 3%.

De manière avantageuse, la composition cosmétique comprend en outre des lipides aptes à restaurer la barrière cutanée, respectivement :
- au moins un lipide exogène de la peau, avantageusement une huile ; et/ou
- un mélange de constituants naturellement présents dans la peau, comprenant des céramides 1, 3, 6, du cholestérol, des acides gras libres et de la phytosphingosine.

En effet, un apport en certains lipides spécifiques permet de restaurer la couche lipidique entre les cornéocytes de la peau, c'est-à-dire le ciment intercellulaire, et donc permet au final la restauration de la barrière cutanée. Il s'ensuit une diminution de l'évaporation de l'eau cutanée, et donc une diminution des risques d'adhésions et de proliférations de *S. aureus* à la surface de la peau. Cette action est combinée avec celles des esters de l'invention qui en inhibant l'adhésion de *S. aureus* empêchent l'hydrolyse des céramides du *stratum corneum.*

Des lipides exogènes de la peau, comme des huiles, permettent une hydratation de la peau à court terme. Ils sont avantageusement choisis dans le groupe comprenant l'huile de tournesol, l'huile de colza (autrement appelée huile de canola), du fait de leur richesse en acides gras essentiels de la série des oméga-6 et des oméga-3.

Dans un mode de réalisation particulier de l'invention, la composition cosmétique comprend des lipides exogènes de la peau, respectivement de l'huile de tournesol, représentant entre 3 et 15% en poids de la composition, et de l'huile de colza (ou canola), représentant entre 0,05 et 10% en poids de la composition.

Un apport en lipides naturellement présents dans la peau permet de combler le déficit en certains lipides dans le *stratum corneum* de la peau atteinte d'atopie cutanée. Ils participent ainsi à restaurer une barrière cutanée fonctionnelle à long terme, et ce en combinaison avec, comme déjà dit l'effet des esters précités.

Dans un mode de réalisation particulier le mélange de constituants naturellement présents dans la peau se présente sous la forme d'une composition lipidique, répondant au nom INCI : Eau, Ceramide 3, Ceramide 6II, Ceramide 1, Phytosphingosine, Cholesterol, Sodium Lauroyl Lactylate, Carbomer, Xanthan Gum. Cette composition lipidique est avantageusement fournie sous la forme du produit commercial SK Influx V™ (Evonik Industries).

De préférence, la composition lipidique représente 0,01 à 5% en poids de la composition.

Selon la présente description, la composition cosmétique comprend en outre au moins un composé polyhydroxylé additionnel choisi dans le groupe comprenant le rhamnose, le xylitol et le mannitol.

Ces composés polyhydroxylés additionnels, comme le rhamnose et le xylitol, contribuent à réduire l'adhésion des bactéries pathogènes, comme *S. aureus*, sur la peau et la muqueuse nasale humaine. Le mannitol, quant à lui, possède une activité antiradicalaire

Dans un mode de réalisation, la composition cosmétique comprend un mélange des trois composés polyhydroxylés additionnels cités ci-dessus.

Selon la présente description, le rhamnose représente entre 0,01 et 1% en poids de la composition, le xylitol représente entre 0,05 et 2% en poids de la composition et le mannitol représente entre 0,005 et 1% en poids de la composition.

La composition peut comprendre en outre au moins un agent antiprurit choisi dans le groupe comprenant notamment :
- le palmitoyl éthanolamide, de numéro CAS 544-31-0 ;
- l'hydroxy-α-sanshool, de numéro CAS 83883-10-7, rentrant dans la composition du zanthalène™ ;
- un lipo-dipeptide à base de tyrosyl-arginine, rentrant dans la composition de la calmosensine™ ;
- l'ichtyol, ou ammonium ichthosulfonate, de numéro CAS 8029-68-3.

La composition cosmétique peut comprendre en outre au moins un agent anti-inflammatoire, préférentiellement choisi dans le groupe comprenant :
- le béta-sitostérol, de numéro CAS 83-46-5 ;
- l'enoxolone, ou acide glycyrrhétinique, de numéro CAS 471-53-4.

La composition cosmétique comprend en outre de la vitamine PP, connue pour stimuler la synthèse des lipides de la couche cornée, telles que les céramides, les acides gras libres et le cholestérol. La vitamine PP agit en stimulant l'activité de la sérine palmitoyl transférase, une enzyme clef de la synthèse de sphingosine, molécule précurseur des céramides.

Un autre aspect de l'invention a pour objet une composition cosmétique pour le traitement cosmétique de l'atopie cutanée.

La présente demande a également pour objet un procédé de traitement cosmétique de l'atopie cutanée consistant à former sur la peau un film protecteur de l'épiderme au moyen de la composition ci-après décrite.

Ainsi, le procédé de traitement consiste à appliquer sur la peau une composition filmogène comprenant notamment un ester de sucrose, inhibant les sites d'adhésion de *S. aureus* à la surface cutanée, et des lipides, par exemple des acides gras, des céramides et du cholestérol, qui renforcent les capacités de défense de la peau vis-à-vis des agressions de microorganismes, et notamment contre les enzymes sécrétées par ces micro-organismes et hydrolysant les composants du *stratum corneum.*

Ce film peut être appliqué en fonction de l'avancement de la pathologie pour :
- limiter l'évolution ;
- agir pendant l'épisode de crise de la maladie ;
- prévenir la récidive.

La composition mise en oeuvre pour limiter l'évolution, comprend, en pourcentage en poids de la composition :
- entre 1 et 5% de sucrose monostéarate;
- entre 3 et 25% d'un mélange d'huile ;
- entre 0,01 et 5% de SK Influx V™ ;
- entre 0,06 et 4% d'un mélange de rhamnose, xylitol, mannitol.

La composition mise en oeuvre pour traiter l'épisode de crise, comprend, en pourcentage en poids de la composition :
- entre 1 et 5% de sucrose monostéarate;
- entre 3 et 25% d'un mélange d'huile ;
- entre 0,01 et 5% de SK Influx V™ ;
- entre 0,06 et 4% d'un mélange de rhamnose, xylitol, mannitol ;
- entre 0,01 et 1% d'un agent antiprurit ;
- entre 0,05 et 1% d'un agent anti-inflammatoire ;

La composition mise en oeuvre, notamment pour prévenir la récidive, comprend, en pourcentage en poids de la composition :
- entre 1 et 5% de sucrose monostéarate;
- entre 3 et 25% d'un mélange huile ;
- entre 0,01 et 5% de SK Influx V™ ;
- entre 0,06 et 3% d'un mélange de rhamnose, xylitol, mannitol ;
- entre 0,01 et 1% d'un agent antiprurit ;
- entre 0,01 et 2% de vitamine PP.

Ces différentes compositions ont un caractère filmogène, c'est-à-dire qu'elles sont aptes à former un film protecteur, à la surface de la peau, vis-à-vis des agressions des microorganismes pathogènes.

Plusieurs avantages ressortent donc à la lecture de l'invention :
- la diminution de l'adhésion de *S. aureus* sur une peau atteinte d'atopie cutanée permet donc d'éviter la prolifération d'une flore de microorganismes pathogènes, tout en maintenant la flore bactérienne saprophyte ;
- il est connu que la formation d'un biofilm bactérien à la surface de la peau est réalisé en plusieurs étapes, dont notamment l'adhésion des bactéries, puis la croissance, la maturation et l'essaimage du biofilm ; ainsi, l'inhibition de l'adhésion de *S. aureus* sur la peau de patient atteint d'atopie cutanée aboutit à l'inhibition de la formation d'un biofilm pathogène ;
- l'utilisation d'un ester de saccharose, non toxique et non irritant, permet de limiter l'emploi d'agents antimicrobiens, comme les antibiotiques et les antiseptiques topiques ;
- la présence de lipides exogènes de la peau permet de restaurer la fonction de barrière et donc de limiter l'évaporation de l'eau contenue dans l'épiderme;
- la complémentarité entre l'ester de saccharose et les lipides permet la diminution du portage de *S. aureus* par le traitement antiadhésion puis la restauration de la fonction de barrière du *stratum corneum* ;
- l'ester de saccharose et les lipides constituent donc sur l'épiderme un film protecteur vis-à-vis des agressions des microorganismes pathogènes afin de réduire les dégradations de la fonction de barrière.

### EXEMPLES

### 1/ EFFET DU SUCROSTEARATE SUR L'INHIBITION IN VITRO DE L'ADHESION DE STAPHYLOCOCCUS AUREUS SUR UN SUPPORT SOLIDE.

### 1) Méthode

Le protocole suivant est mis en oeuvre :
- Une préculture de *S. aureus* issue du laboratoire de bactériologie clinique de l'hôpital de la Timone (CIP 65-8) est réalisée dans du milieu 'Nutrient Broth N° 2' (Oxoid), pendant 12h, à 37°C ;
- La culture est centrifugée à 9 000 g, pendant 10 min et le culot bactérien est remis en suspension dans un tampon phosphate (PBS) ;
- A partir de cette suspension, une dilution au 1/50 est réalisée dans une solution aqueuse ou une solution aqueuse contenant le composé polyhydroxylé à tester. Il s'agit soit du xylitol, soit du sucrostéarate HLB 16 (SURFHOPE™ C1816) ;
- Les bactéries sont préincubées en présence du composé polyhydroxylé à tester pendant 45 min, à 37 °C ;
- Le mélange (100 µl) est ensuite déposé dans un puits d'une lame cytologique en verre, de surface fixe (0,8 cm²) et délimitée par des parois, c'est-à-dire une lame adaptée à la culture cellulaire (Chamber Slide System, Labtek) ;
- Les cultures bactériennes sont ensuite incubées à 37°C, pendant 60 min et ultérieurement rincées délicatement avec de l'eau ultra pure, ce qui permet d'éliminer les bactéries n'ayant pas adhéré à la surface du puits de la lame ;
- Les bactéries ayant adhéré dans chaque puits sont par la suite colorées par le cristal violet pendant 10 secondes, puis rincées et séchées pendant 12h, à 25°C ;
- Les bactéries sont enfin observées à l'aide d'un microscope optique, par grossissement x 1000, en immersion, les champs étant parfaitement homogènes ;
- Un comptage automatique des bactéries dans chaque puits est finalement réalisé à l'aide d'un logiciel approprié.

### 2) Résultats

Selon ce protocole, l'effet des composés polyhydroxylés a été analysé pour l'activité d'inhibition de l'adhésion de *S. aureus* sur le support solide. Le xylitol à une concentration finale de 0,5% et le sucrostéarate HLB 16 (sucrose monostéarate, SURFHOPE C18-C16) à une concentration finale de 1% ont été testés.

Tableau 1 : Effets du xylitol (0,5%) et du sucrostéarate HLB 16 (1%) sur l'adhésion de *S. aureus* à la surface des lames de verre. Les résultats correspondent à une moyenne du comptage de trois champs, qui est présentée avec l'écart type correspondant.

| **Condition** | **Nombre de cellules par champ** | **inhibition de l'adhésion (% par rapport au témoin eau)** |
|---|---|---|
| **Eau (témoin)** | 99 ± 11 | 0% |
| **Xylitol (0,5%)** | 91 ± 6 | 8% |
| **Sucrostéarate HLB 16 (1%)** | 12 ± 1 | 88% |

### 3) Conclusion

Une forte inhibition de l'adhésion de *S. aureus* à la surface des lames de verre est observée en présence de 1% sucrostéarate HLB 16.

### 2/ EFFET DES ESTERS DE L'INVENTION SUR L'INHIBITION EX VIVO DE L'ADHESION DE S. AUREUS SUR LES CORNEOCYTES HUMAINS.

Afin de corroborer les résultats obtenus *in vitro,* un test d'adhésion *ex vivo* sur des cornéocytes humains a été mis au point d'après la méthode réalisée chez le chien (McEwan *et al.,* 2005).

### 1) Souche bactérienne

La souche clinique de *S. aureus* (CIP 65-8) a été prélevée sur un patient, au sein du laboratoire de bactériologie clinique de l'hôpital de la Timone à Marseille.

### 2) Protocole expérimental

La souche de *S. aureus* est inoculée dans 10 ml de milieu 'Nutrient Broth N° 2' (Oxoid) et incubée sous agitation à 37°C pendant 12 heures. Les bactéries sont collectées par centrifugation et le culot bactérien est remis en suspension dans un tampon phosphate PBS. La population bactérienne est ajustée approximativement à 10⁶ CFU/ml avec de l'eau osmosée.

Des zones cibles sont délimitées sur chaque bras d'un volontaire humain. Pour chaque zone, les débris cutanés sont éliminés grâce à 5 « pré-strippings » successifs avec des disques Sellotape™Original (diamètre 22 mm). Les cornéocytes sont ensuite collectés sur chaque zone cible à l'aide de disques adhésifs D-Squame™ (diamètre 22 mm) et d'un applicateur de disque D-Squame réglé à la pression de 150 g/cm².

Les disques sont ensuite placés dans des boîtes de Pétri, de diamètre 35 mm. Les disques sont alors recouverts par 0,5 ml d'eau ou 0,5 ml de la solution de *S. aureus* à 10⁶ CFU/ml, contenant le composé polyhydroxylé à tester, à raison de trois zones pour chacun d'entre eux.

Les composés polyhydroxylés testés avec ce protocole expérimental sont :
- le xylitol, à une concentration finale de 0,5% ;
- le sucrose stéarate HLB 16 (sucrose monostéarate, SURFHOPE C1816,), à une concentration finale de 1%,
- le sucrose laurate monoester (SURFHOPE C1216), à une concentration finale de 0,1%,

Toutes les solutions sont préparées dans de l'eau et mises en contact avec *S. aureus* à température ambiante 45 min avant le début de l'expérience d'adhésion. Les boites sont ensuite incubées à 37°C pendant 60 min. Les disques sont alors rincés avec de l'eau osmosée, ce qui permet d'éliminer les bactéries n'ayant pas adhéré aux cornéocytes. Les bactéries ayant adhéré aux cornéocytes humains sont alors colorées par du cristal violet oxalaté pendant 10 secondes. Les disques sont enfin rincés avec de l'eau osmosée, placés sur une lame de microscope et séchés à l'air libre pendant 24 h.

### 3) Acquisition des images et comptage des bactéries

Les images des bactéries adhérentes sont acquises à l'aide d'un microscope Olympus BX 53 couplé à une caméra numérique. Les images collectées sont ensuite analysées par le logiciel UTHSCSA Image Tool (Reindeer Graphics Inc.). Le nombre de bactéries par disque est calculé par une moyenne arithmétique de 10 à 12 images, ce qui correspond à la surface d'environ 100 cornéocytes. Les comparaisons entre différentes moyennes sont effectuées avec le logiciel Statgraphics Plus (Manugistics Inc.).

### 4) Résultats et conclusion

Les résultats de ces tests d'inhibition de l'adhésion de *S. aureus* montrent que le xylitol a un effet inhibiteur sur l'adhésion de *S. aureus* sur les cornéocytes d'environ 20% (Tableau 2). Une très forte activité inhibitrice supérieure ou environ égale à 50% est obtenue pour le sucrostéarate de HLB égal à 16, utilisés à une concentration finale de 1% (Tableau 2). En revanche, les résultats montrent que le sucrose laurate monoester entraine au contraire une augmentation significative de l'adhésion.

**Tableau 2 : Effet du xylitol et des sucres de l'invention sur l'inhibition de l'adhésion de S. aureus sur les cornéocytes humains.**

| | **Inhibition de l'adhésion de *S. aureus* (%)** | |
|---|---|---|
| **Composé polyhydroxyle (concentration finale)** | Essai 1 | Essai 2 |
| Xylitol (0,5%) | 21,0 | 20,2 |
| Sucrostéarate HLB16 (1%) | 57,0 | 49,6 |
| Sucrose laurate (0,1%) | -22.8 | |

Chaque sucre a été testé dans deux essais indépendants pour xylitol et sucrose stéarate (1%).

Ces résultats *ex vivo* confirment donc les résultats obtenus *in vitro,* à savoir que la présence de sucrostéarate HLB 16 dans l'environnement de *S. aureus* a une action inhibitrice sur son adhésion, tant au niveau d'un support solide comme une lame cytologique, que sur des cornéocytes humains.

### 3/ EXEMPLES DE COMPOSITIONS TOPIQUES

**ATODERM™ STOP EVOLUTIVE**

| **Produit** | **% en poids dans la composition** |
|---|---|
| Sucrostéarate HLB 16 (SURFHOPE C1816) | 2,0 |
| Phase lipidique | 11,0 |
| Autres composés polyhydroxylés | 0,61 |
| Eau | Qsp 100 |

**ATODERM™ STOP CRISE**

| **Produit** | **% en poids dans la composition** |
|---|---|
| Sucrostéarate HLB16 (SURFHOPE C181)6 | 2,0 |
| Phase lipidique | 11,0 |
| Autres composés polyhydroxylés | 0,61 |
| Agent antiprurit | 0,3 |
| Agent anti-inflammatoire | 0,5 |
| Eau | Qsp 100 |

**ATODERM™ STOP RECIDIVE**

| **Produit** | **% en poids dans la composition** |
|---|---|
| Sucrostéarate HLB16 (SURFHOPE C1816) | 2,0 |
| Phase lipidique | 11,0 |
| Autres composés polyhydroxylés | 1,1 |
| Agent antiprurit | 0,3 |
| Vitamine PP | 0,3 |
| Eau | Qsp 100 |

### REFERENCES

Kita K., Sueyoshi N., Okino N., Inagaki M., Ishida H., Kiso M., Imayama S., Nakamura T., Ito M. 2002. Activation of bacterial ceramidase by anionic glycerophospholipids : possible involvement in ceramide hydrolysis on atopic skin by Pseudomonas ceramidase. Biochem J., 362, p. 619-626.
McEwan N.A., Kalna G. and Mellor D. 2005. A comparison of adherence by four strains of Staphylococcus intermedius and Staphylococcus hominis to canine corneocytes collected from normal dogs and dogs suffering from atopic dermatitis. Research in Veterinary Science, Vol. 78, p. 193-198.
Piccicuto S., Blecker C., Brohée J.C., Mbampara A., Lognay G., Deroanne C., Paquot M. and Marlier M. 2001. Les esters de sucres : voies de synthèse et potentialités d'utilisation. Biotechnol. Agron. Soc. Environ., Vol. 5(4), p. 209-219.
Pascolini C., Sinagra J., Pecetta S., Bordignon V., De Santis A., Cilli L., Cafiso V., Prignano G., Capitanio B., Passariello C., Stefani S., Cordiali-Fei P., Ensoli F. 2011. Molecular and immunological characterization of Staphylococcus aureus in pediatric atopic dermatitis: implications for prophylaxis and clinical management. Clin Dev Immunol. 2011 Oct 27 (Epub).

## Revendications

1. Composition pour application topique comprenant :
- au moins un sucrose stéarate comprenant au moins 75% en poids de monoesters de sucrose et d'acide stéarique, le complément étant composé de di, tri, polyesters de sucrose et d'acide stéarique et présentant une HLB de 16 (sucrose monostéarate), ,
pour utilisation dans le traitement thérapeutique de l'atopie cutanée comme agent inhibant l'adhésion de *Staphylococcus aureus* sur la peau et la muqueuse nasale humaine.

2. Composition pour son utilisation selon la revendication 1, **caractérisée en ce que** le sucrose stéarate représente 0,1 à 5% en poids de la composition, avantageusement entre 1 et 3%.

3. Composition pour son utilisation selon la revendication 1 ou 2, **caractérisée en ce qu'**elle comprend en outre des lipides aptes à restaurer la barrière cutanée, respectivement :
- au moins un lipide exogène de la peau, avantageusement une huile ; et/ou
- un mélange de constituants naturellement présents dans la peau, comprenant des céramides 1, 3, 6, du cholestérol, des acides gras libres et de la phytosphingosine.

4. Composition pour son utilisation selon la revendication 3, **caractérisée en ce que** le mélange de constituants naturellement présents dans la peau se présente sous la forme d'une composition lipidique, répondant au nom INCI : Eau, Ceramide 3, Ceramide 6II, Ceramide 1, Phytosphingosine, Cholesterol, Sodium Lauroyl Lactylate, Carbomer, Xanthan Gum, et représente entre 0,01 et 5% en poids de la composition.

## Patentansprüche

1. Zusammensetzung zur topischen Anwendung, umfassend:
- mindestens ein Saccharosestearat, umfassend mindestens 75 Gewichts-% Saccharosemonoester und Stearinsäure, wobei der Rest aus Di-, Tri- und Polyestern von Saccharose und Stearinsäure besteht, mit einem HLB-Wert von 16 (Monostearat- Saccharose),
zum Einsatz bei der therapeutischen Behandlung der kutanen Atopie als Wirkstoff, der die Adhäsion von *Staphylococcus aureus* auf der Haut und Nasenschleimhaut des Menschen verhindert.

2. Zusammensetzung zur Anwendung gemäß Anspruch 1, **dadurch gekennzeichnet, dass** das Saccharosestearat 0,1 bis 5% Gewichts- % der Zusammensetzung am besten zwischen 1 und 3 % umfasst.

3. Zusammensetzung zur Anwendung gemäß Anspruch 1 oder 2, **dadurch gekennzeichnet, dass** sie außerdem Lipide enthält, die in der Lage sind, die Hautbarriere wiederherzustellen, und zwar:
- mindestens ein hautexogenes Lipid, am besten ein Öl; und/oder
- eine Mischung aus Bestandteilen, die in der Haut natürlich vorhanden sind, die die Cholesterinceramide 1, 3, 6, freie Fettsäuren und Phytosphingosin umfassen.

4. Zusammensetzung zur Anwendung gemäß Anspruch 3, **dadurch gekennzeichnet, dass** die Mischung aus natürlichen Hautbestandteilen die Form einer Lipid- Zusammensetzung mit dem Namen INCI: Wasser, Ceramid 3, Ceramid 611, Ceramid 1, Phytosphingosin, Cholesterin, Natrium-Lauroyl-Lactylat, Carbomer, Xanthan-Gummi hat und sie zwischen 0,01 und 5 Gewichts-% der Zusammensetzung bildet.

## Claims

1. Composition for topical application, containing:
- at least a sucrose stearate containing at least 75% monoesters of sucrose and stearic acid by weight, the rest being di, tri and polyesters of sucrose and stearic acid, with a HLB of 16 (sucrose monostearate),
for use in the therapeutic treatment of atopic dermatitis as an agent to inhibit the adhesion of *Staphylococcus aureus* on human skin and nasal mucosa.

2. Composition for its use according to Claim 1, **characterised in that** the sucrose stearate represents 0.1 to 5% of the composition by weight, advantageously between 1% and 3%.

3. Composition for its use according to Claim 1 or 2, **characterised in that** it also contains lipids that can restore cutaneous barrier function, respectively:
- at least one lipid not found naturally in the skin, advantageously an oil; and/or
- a mixture of constituents that occur naturally in the skin, including ceramides 1, 3, 6, cholesterol, free fatty acids and phytosphingosine.

4. Composition for its use according to Claim 3, **characterised in that** the mixture of constituents that occur naturally in the skin corresponds to a lipid composition with an INCI designation, namely: Water, Ceramide 3, Ceramide 6II, Ceramide 1, Phytosphingosine, Cholesterol, Sodium Lauroyl Lactylate, Carbomer, Xanthan Gum, representing between 0.01% and 5% of the composition by weight.
